**Europäisches Patentamt**

**European Patent Office**

**Office européen des brevets**

(19)

(11) Publication number: **0 117 072**
**A1**

(12) # EUROPEAN PATENT APPLICATION

(21) Application number: **84300383.1**

(22) Date of filing: **23.01.84**

(51) Int. Cl.³: **A 61 F 1/00**

(30) Priority: **25.01.83 GB 8302003**

(43) Date of publication of application:
**29.08.84 Bulletin 84/35**

(84) Designated Contracting States:
**AT BE CH DE FR GB IT LI LU NL SE**

(71) Applicant: **J. & P. Coats, Limited**
**155 St. Vincent Street**
**Glasgow G2 5PA Scotland(GB)**

(72) Inventor: **Hood, Robert Gordon**
**"Koinonia" 30 Park Avenue**
**Paisley Renfrewshire Scotland(GB)**

(54) Vascular prosthesis.

(57) A vascular prosthesis to be used as a graft for replacing a portion of a blood vessel is in the form of a knitted tubular structure (1) the wall (2,3) of which comprises several strata (4,5,6) of which each stratum has characteristics different from those of at least one of the other strata.

The prosthesis provides specific characteristics by determining individually the characteristics of each stratum (4,5,6).

Croydon Printing Company Ltd.

EP 0 117 072 A1

-1-

## Vascular Prosthesis

The subject of this invention is a vascular prosthesis and particularly a prosthesis in the form of a knitted tube to be used as a graft for replacing a portion of a blood vessel in a human or animal body.

In the formation of a prosthesis formed as a substitute blood vessel it is essential that various characteristics of the prosthesis should be predeterminable. One of the most important characteristics to be predetermined is the degree of porosity. The pores of such a prosthesis must be of a size to suit the particular medical condition of the proposed recipient of the prosthesis. There are other characteristics which should also be predetermined, for example, the relative smoothness of the surfaces of the prosthesis, the density of the structure and its flexibility. As in the case of porosity, the degree of relative smoothness of the surface and the density are dictated by the particular degree of tissue response required of the respective surfaces of the prosthesis after implantation. Varying degrees of flexibility are required to ensure that on implantation the prosthesis will conform to the anatomical situation at the site of implantation.

Hitherto in the manufacture of knitted vascular prostheses it has not been possible to make significant changes to any one of these characteristics without adversely affecting one or more of the others. It would be of great advantage in the manufacture of knitted vascular prostheses if it were possible to vary substantially independently, all the characteristics of a prosthesis referred to above because at present, by the known methods of manufacture, the characteristics of such prostheses
are to a large degree a matter of compromise. A
further disadvantage present in known knitted vascular
prostheses is that it has been necessary, prior
to implantation, to "pre-clot" the prosthesis to
be implanted. By the expression "pre-clot" or
"pre-clotting" is meant the step during surgery
on the recipient of soaking the prosthesis in blood,
normally the patient's blood or in certain circumstances
donor blood of the same group until clot formation
occurs on the surface of the prosthesis. In the
implantation of known knitted vascular prostheses
such pre-clotting reduces the amount of blood lost
by seepage through the knitted structure after implantation. The step is necessary because known knitted
vascular prostheses cannot exhibit a degree of porosity
low enough to avoid serious blood loss after implantation.
It would be a significant advance if the operating
time and loss of blood by the patient necessarily
associated with the pre-clotting step could be
eliminated or significantly reduced. It is an
object of the present invention to provide a vascular
prosthesis in which the various characteristics
are independently predeterminable to a much greater
extent than has been the case heretofore. It is
a further object of this invention to provide a
knitted vascular prosthesis having interstices of
a pre-determinable size small enough to reduce blood
loss during the surgical procedure but large anough
to allow cell migration through the structure of
the prosthesis after implantation and which allows
the pre-clotting step to be reduced or in certain
cases eliminated.

A vascular prosthesis according to the
invention comprises an integrated knitted tubular
structure in which the wall of the tube is composed of several strata of which each stratum has characteristics different from those of at least one of the other strata.

The prosthesis may be knitted from several yarns at least two of which have specific characteristics different from one another with one chosen yarn in preponderance in the stratum presenting the outer surface of the tubular structure and another chosen yarn in a preponderance in the stratum presenting the inner surface of the tubular structure and at least one other yarn forms at least one stratum between the other two strata.

In one example of such a prosthesis formed of three yarns two of the yarns are formed of polyester and one is formed of polytetrafluoroethane usually referred to as PTFE, the knit being such that polyester yarn forms the stratum providing the preponderance of polyester at the outer surface and the PTFE yarn forms the stratum providing the preponderance of PTFE at the inner surface. Other yarns which may be used to form particular strata are carbon fibres, polypropylene, polyurethane and some grades of natural rubber, also yarns chosen from the class of yarns known as spandex yarns.

It has been found that three yarns are effective in most situations in making a prosthesis with chosen characteristics but it is to be understood that more than three yarns may be used in special circumstances.

A method of manufacturing a prosthesis according to the invention comprises feeding several yarns to the needles of a knitting machine of the two needle bed type with the chosen yarns being fed to the needles of one bed and the other yarn or yarns being fed to the needles of the other bed.

Different yarns fed to the same needle bed may be fed with different tensions, the tensions in the different yarns fed to the same needle being such that the yarn to be in preponderance in the surface stratum to be formed by that needle bed is the yarn fed with the least tension.

In one particular method of manufacture separate stratified flat structures are knitted simultaneously back to back and are knitted together along the selvedges to form the required tubular structure. This operation may be performed in a knitting machine with two double needle beds arranged back to back, the knitting operation being performed immediately the two strips are formed on their own needle beds.

The finished tubular structure may be formed with circumferential corrugations.

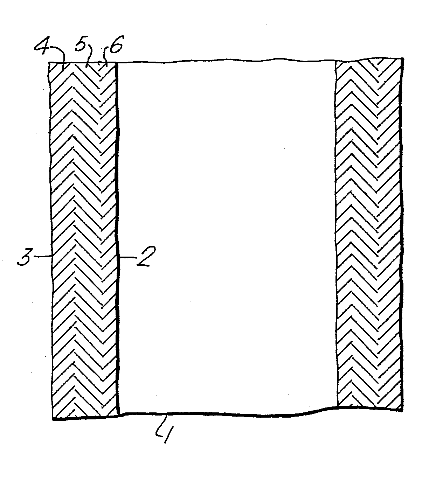

A diagrammatic longitudinal section to a greatly enlarged scale through a vascular prosthesis according to the invention is illustrated in the accompanying drawing which shows a tubular structure in the form of an uncorrugated tube. In the drawings 1 denotes a tube having an inner wall surface 2 and an outer wall surface 3. The wall itself presents three integrated strata 4, 5 and 6 which merge with one another without any definite dividing line between them. The three strata are formed from three yarns knitted together with different tensions as already described to provide that the stratum 4 consists of the material which preponderates on the outer surface e.g. polyester, the stratum 6 is the stratum in which the inner surface material preponderates e.g. PTFE and the stratum 5 contains the material of the third yarn mixed with a certain amount of the material of the yarn of the stratum 4 and the yarn of the stratum 6. The different tensions for the yarns forming these strata are chosen to give the desired characteristics to these strata and the desired proportions of the particular materials required for these strata. By varying the proportions of the tension one to the other it is possible to vary the overall characteristics for example of porosity of the tubular structure while the characteristics of the individual strata can also be varied to give specific characteristics according to the positions of the strata in the tubular structure itself. By this means it is possible to make a composite tubular structure which satisfies a large number of exacting chosen requirements. By existing methods of manufacture that has not been heretofore possible and known grafts have all been to some extent a compromise in their characteristics.

CLAIMS

1. A vascular prosthesis having an integrated knitted tubular structure characterized in that the wall (2,3) of the tubular structure is composed of several strata (4,5,6) each stratum having characteristics different from those of at least one of the other strata.

2. A vascular prosthesis according to claim 1 characterized in that the prosthesis is knitted from several yarns at least two of which have specific characteristics different from one another with one chosen yarn in preponderance in the stratum (4) presenting the outer surface of the tubular structure and another chosen yarn in a preponderance in the stratum (6) presenting the inner surface of the tubular structure and that at least one other yarn forms at least one stratum (5) between the other two strata.

3. A vascular prosthesis according to claim 2 characterized in that two strata are of knitted polyester yarn and a third stratum is of knitted polytetrafluoroethane yarn, the knit being such that polyester yarn forms the stratum (4) providing the preponderance of polyester at the outer surface (3) and the polytetrafluoroethane yarn forms the stratum (6) providing the preponderance of polytetrafluoroethane at the inner surface (2).

4. A method of manufacturing a vascular prosthesis having an integrated knitted tubular structure the tubular wall (2,3) of which is composed of several strata (4,5,6) knitted from several yarns at least two of which have specific characteristics different from one another with one chosen yarn in preponderance in the stratum presenting the outer surface (3) of the tubular structure and another chosen yarn in preponderance in the stratum presenting the inner surface (2) of the tubular structure and at least one other yarn forming at least one stratum (5) between the other two strata, characterized by feeding the said chosen yarns to the needles of one bed of a knitting machine of the two needle bed type and feeding the other yarn or yarns to the needles of the other bed of the knitting machine.

5. A method of knitting a vascular prosthesis according to claim 4, characterized in that different yarns fed to the same needle bed are fed with different tensions.

6. A method of knitting a vascular prosthesis according to claim 4 characterized by knitting simultaneously and back to back two flat strips composed of several strata of different characteristics and knitting the strips together along their selvedges to form a tubular structure.

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| Y | US-A-4 047 252 (LIEBIG et al.) * Claim 1 * | 1 | A 61 F 1/00 |
| Y | WO-A-8 201 647 (KASTER) * Claims 1, 18-20, 22-26; page 8, lines 24-26 * | 1 | |
| Y | US-A-3 105 492 (JECKEL) * Claims 1-3; column 2, lines 57-61 * | 1 | |
| A | DE-A-2 461 370 (SAUVAGE et al.) | | |
| A | WO-A-8 002 641 (BOWALD et al.) | | |
| A | DE-A-3 019 996 (INSTITUTE FÜR TEXTIL- UND FASERFORSCHUNG) | | **TECHNICAL FIELDS SEARCHED (Int. Cl. ³)** A 61 F 1/00 |
| A | US-A-3 945 052 (LIEBIG) | | |

The present search report has been drawn up for all claims

| Place of search BERLIN | Date of completion of the search 11-04-1984 | Examiner KANAL P K |
|---|---|---|

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82